# EUROPEAN PATENT APPLICATION

(11) **EP 2 821 056 A1**
(43) Date of publication of application: **07.01.2015**
(21) Application number: 14175168.5
(22) Date of filing: 01.07.2014
(51) Int. Cl.: A61K 8/49, A61Q 19/06, A61K 8/97

(54) **Cosmetic composition, formulation gel-cream and its use**

(30) Priority: 01.07.2013 BR 102013016991
(71) Applicant: EMS S.A., 13186-901 Sao Paulo - SP (BR)
(72) Inventor: Masiero, Silvana, 13186-901 Sao Paulo - SP (BR); Magalhaes Nadaluti, Rebeca, 13186-901 Sao Paulo - SP (BR); Khater Covesi, Letícia, 13186-901 Sao Paulo - SP (BR); Gobbo Chaves, Fernanda, 13186-901 Sao Paulo - SP (BR); Maria Barbosa, Juliana, 13186-901 Sao Paulo - SP (BR)
(74) Representative: Cabinet Plasseraud

(57) **Abstract**

The present invention aims to provide reduction of localized fat, particularly for the treatment, prevention or alleviation of gynoid lypodystrophy.

The cosmetic composition of the invention comprises:
(a) from 4.0 to 27.0% by weight of active ingredients comprising:
(i) at least one localized fat reducing agent selected from the group consisting of natural, modified and synthetic substances,
(ii) at least one alkaloid,
(iii) at least one oil or vegetable extract,
(iv) at least one antioxidant, and
(v) at least one film forming agent;

(b) 6.0 to 32.0% by weight of cosmetically acceptable additives; and
(c) q.s.p. 100% by weight of hydrophilic gel-cream base.

The invention also comprises a gel-cream formulation for localized fat reduction. Finally, the invention is also directed to said composition of gel-cream formulation for the treatment, prevention or alleviation of gynoid lypodystrophy.

## Description

### Field of the Invention

The present invention relates to a cosmetic composition for the reduction of localized fat, particularly for the treatment, prevention or alleviation of gynoid lipodystrophy, said composition comprising at least one fat-reducing agent, for example, a compound formed by silanol, acefylline and alginic acid and/or a combination of an ester of salicylic acid (salicylamide) in polyoxyethylene, an alkali compound, an oil and/or plant extract, a natural antioxidant, a film-forming polymer and cosmetically acceptable additives, said composition being in the form a gel-cream. The invention is also directed to a cosmetic product comprising said composition, preferably said cosmetic product being a gel-cream. The invention also comprises the composition/formulation for the treatment or prevention of gynoid lipodystrophy.

### Background of the Invention

The term cellulite was first described in 1920 (Scherwitz C, Braun-Falco O. So-called cellulite. J Dermatol Surg Oncol. 1978; 4 (3): 230-4; Rossi B & Vergnan ini AL. Cellulite: a review. J Eur Acad Dermatol Venereol 2000, 14(4):251-62; Avram MM. Cellulite: a review of its physiology and treatment. J Cosmet Laser Ther 2004, 6 (4): 181:5; Ciporkin H & Pascoal LH. Therapeutic update and physiopathology of gynoid lipodystrophy (LDG) "Cellulite." Sao Paulo: Santos, 1992). The word, Cellulite, of Latin origin, was used to describe a cosmetic change to the skin surface. Cellulite is not the most appropriate term, because the derivation of the word means inflammation, and cell studies suggest that no signs of inflammation have been found in the tissue in question.

Despite being an incorrect designation, the term cellulite is currently accepted and describes a benign condition known by various medical terms. There are several terms used to define these changes in the subcutaneous tissue, in an attempt to suit the histomorphological changes, namely: Lipodystrophy, Lipoedema, Fibro Edema geloid, Hydrolipodistrophy, Gynoid Hydrolipodystrophy, Panniculopathy edematous-fibrous sclera, Paniculose, Nodular Lipoesclerosis, Gynoid Lipodystrophy and Fibrosclerotic Dermatomyositis, among others (Giménez AM. Celulitis. Um problema cosmético controvertido. Act Dermatol. 2001; 40: 595-610; Schneider AP. Nutrição estética. São Paulo: Atheneu; 2010.p.168-9; Avram MM.Cellulite: a review of its physiology and treatment. J Cosmet Laser Ther. 2004; 6(4):181-5; Terranova F, Berardesca E, Maibach H. Cellulite: nature and aetiopatho- genesis. Int J Cosmet Sci. 2006; 28(3):157-67; Lotti T, Ghersetich I, Grappone C, Dini G. Proteoglycans in so-called cellulite. Int J Dermatol.1990; 29(40:272-4; Rossi ABR, Vergnanini AL. Cellulite: a review. J Eur Acad Dermatol. Venereol 2000;14(4): 251-62).

We highlight the Hydrolipodystrophy terminology, which defines a pathological change of the hypodermis (Lipodystrophy) with edema (Hydro) and alteration of veno-lymphatic function.

Lipodystrophy is a pathological change; a syndrome caused by loss and/or destruction of adipocytes. The disorder is characterized by a selective loss of fat and is associated with hypertriglyceridemia, hepatic steatosis, among others (Rossini et al.1977, Metabolism, 26: 637-650; Reitman et al. 2000, Metab. Endocrinol. Trends,11:410-416; Arioglu et al. 2000, Ann. Intern. Med. 133: 263-274).

Gynoid Lipodystrophy can be a cosmetic problem characterized by the presence of small depressions in the skin, with the aspect known as orange peel, and occurs in 90% of women after adolescence and rarely occurs in men. The condition appears to be hereditary, usually appearing after puberty with the highest incidence after the age of 30, located mainly on the buttocks, thighs and abdomen.

Cellulite occurs mainly in women, in the pelvic region, lower limbs and abdomen, and can affect any part of the body except the scalp, palms of the hands and feet. It is characterized by a quilted or "orange peel" appearance. The condition is also defined by some authors as a metabolic disorder located in the subcutaneous tissue, which causes a change in the shape of the female body.

It is estimated that cellulite affects most women. This condition is not properly considered a pathological condition, but can cause aesthetic and psychosocial disturbances which may be caused by current aesthetic standards. However, considering reports from the World Health Organization, an individual is a healthy balance of a biopsychosocial framework. Thus, Hydrolipodystrophy can be considered a health problem.

Although this clinical picture also occurs in patients with cellulitis, there are several structural changes in the dermis and microcirculation.

The exact etiology of cellulite is unknown. For some authors it is characterized as a complex cellular dystrophy, accompanied by changes of water metabolism, resulting in a saturation of the connective tissue.

The authors suggest that there are three main hypotheses: anatomical and hormonal changes, chronic inflammation and microcirculation.

The anatomical hypothesis of cellulite is based on differences between men and women in relation to the structural characteristics of the lobules of subcutaneous fat and connective tissue septa that separate them. According to this theory, originally detailed by Muller and Nurnberger (Nurnberger F, Muller G. So-called cellulite: an invented disease. J Dermatol Surg Oncol.1978; 4(3):221-9), the appearance of cellulite, characterized by a wavy and uneven appearance of the skin is caused by the protrusion of fat in the dermahypodermic interface. This change occurs specifically in women due to the presence of vertical fascial bands (Avram MM. Cellulite: a review of its physiology and treatment. J Cosmet Laser Ther. 2004; 6 (4):181-5; Nurnberger F, Muller G. So-called cellulite: an invented disease. J Dermatol Surg Oncol. 1978; 4(3):221-9; Alster TS, Tehrani M. Treatment of cellulite with optical devices: an overview with practical considerations. Lasers Surg Med 2006; 38(8):727-30). Some authors believe the hypothesis that cellulite is a result of stretching of these fascial bands which are genetically determined (Piérard GE, Nizet JL, Pierard-Franchimont C. Cellulite: from standing fat herniation to hypodermal stretch marks. Am J Dermatopathol. 2000; 22 (1): 34-7). The stretching, in turn, weakens and thins the base of the dermal connective tissue allowing the protrusion of fat in the dermo-hypodermic interface, causing an irregular and wavy appearance of the skin. These herniations of fat in the dermis are characteristic of the female anatomy, and its presence has been confirmed by ultrasound in low density regions interspersed in the dermis (M Rosenbaum et al. An exploratory investigation of the morphology and biochemistry of cellulite. Plast Reconstr Surg. 1998; 101 (7): 1934-9; Callaghan T & Wilhelm KP. An examination of non-invasive imaging techniques in the analysis and review of cellulite. Int J Cosmet Sci 2006; 28(3):231; ZD. The disease of cellulite. J Cosmet Dermatol. 2005; 4:221-2). The subcutaneous layer in men, on the other hand, is characterized by horizontal and diagonal fascial bands forming a structure which prevents fat herniation (AS Avram et al. Subcutaneous fat in normal and diseased states II (anatomy and physiology of white and brown adipose tissue). J Am Acad Dermatol. 2005; 53(4:671-9).

Since cellulite is present largely in post pubertal women and is rarely found in men without androgen deficiency, it is highly likely that female hormones play a key role in its pathogenesis (Wanner M & Avram M. An evidence-based assessment of treatments for cellulite. J Drugs Dermatol. 2008; 7(4:341-5). It is because of the hormonal and genetic nature of skin architecture that cellulite is extremely rare in men with normal androgen levels, regardless of weight (Alster TS & Tehrani M. Treatment of cellulite with optical devices: an overview with practical considerations. Lasers Surg Med 2006; 38(8):727-30). In a study of ultrasound to examine the total thickness of the skin of affected and unaffected areas of the thigh, with subsequent wedge biopsy, research tried to determine whether or not the skin dimpling resulted from dermal fat herniations. (Nurnberger F & G. Muller So-called cellulite: an invented disease. J Dermatol Surg Oncol.1978; 4(3):221-9). To achieve this, seven healthy adult women with cellulite and three healthy unaffected individuals (controls), consisting of one woman and two men, were examined. Both the affected women, and the unaffected women, demonstrated irregular and discontinuous dermo-hypodermal interface, characterized by the protrusion of fat into the dermis. The dermo-hypodermic transition in men studied was smooth and continuous. (Avram MM Cellulite: A review of its physiology and treatment. J Cosmet Laser Ther. 2004; 6(4):181-5).

While there are several assumptions to explain the appearance of cellulite, the best of them points to the hormonal differences as being responsible for structural changes in the architecture of subcutaneous fat of women, i.e., it is a fundamentally anatomical change (Wanner M & Avram M. An evidence -based assessment of treatments for cellulite. J Drugs Dermatol. 2008; 7(4):341-5).

The alternative of vascular changes was raised in a review article, by Rossi and Vergnanini, which described the multifactorial basis for the etiology of cellulite (ABR Rossi, AL Vergnanini Cellulite: A review. J Eur Acad Dermatol. Venereol 2000; 14(4):251-62). Supported in the descriptions of Curri (SB Curri. Cellulite and fatty tissue microcirculation. Cosmet Toilet. 1993; 108:51-8; Curri SB, Bombardelli E. Local lipodystrophy and districtual microcirculation. Cosmet Toilet. 1994; 109:51-65) Structural and metabolic events were detailed. According to this theory, the process would originate with the deterioration of the cutaneous vasculature, particularly in response to changes in the arteriolar precapillary sphincter in affected areas, along with the deposition of hyperpolimerized glycosaminoglycans in the wall of dermal capillaries and between the collagen and elastic fibers (Avram MM Cellulite: a review of its physiology and treatment. J Cosmet Laser Ther. 2004; 6(4):181-5; Alster TS, Tehrani M. Treatment of cellulite with optical devices: an overview with practical considerations. Lasers Surg Med 2006;38(8):727-30; Querleux B, Cornillon C, Jolivet O, Bittoun J. Anatomy and physiology of subcutaneous adipose tissue by in vivo magnetic resonance imaging and spectroscopy: Relationships with sex and presence of cellulite. Skin Res Technol. 2002; 8 (2):118-24). The increase in capillary pressure would lead to increased capillary venular permeability and retention of excess fluid in the dermis, adipocytes and between the inter-lobular septa, causing cellular changes and tissue hypoxia.

Increased lipolytic resistance resulting from hypoxia and increased lipogenesis, caused by the action of estrogen, prolactin and high carbohydrate diets leads to adipocyte hypertrophy. The enlarged adipocytes, along with hypertrophy and hyperplasia of the adipocyte peri reticular fibers, would form micronodules surrounded by protein fragments which subsequently cause sclerosis of fibrous septa, leading to the appearance of cellulite. The overall effect of this pathophysiological process is a reduction of blood flow and lymphatic drainage of the affected areas. In accordance with this theory, many therapies have become popular, trying to fix the tissue circulation and inefficient drainage, with the goal of improving the appearance of wavy and uneven skin (Alster TS, Tehrani M. Treatment of cellulite with optical devices: an overview with practical considerations. Lasers Surg Med 2006; 38(8):727-30).

Some authors suggest a third hypothesis, based on the inflammatory pathophysiology of cellulite which relies on subjective reports about their understanding (Draelos Z, Marenus KD. Cellulite etiology and purported treatment. Dermatol Surg. 1997; 23(12):1177-81; Scherwitz C, Braun-Falco O. So-called cellulite. J Dermatol Surg Oncol. 1978; 4 (3): 230-4; Collis N, Elliot LA, Sharpe C, Sharpe DT. Cellulite treatment: a myth or reality: a prospective randomized, controlled trial of two therapies, endermologie and aminophylline cream. Plast Reconstr Surg. 1999; 104(4):1110-4). Kligman (Kligman AM Cellulite:. Facts and fiction. Geriatric J Dermatol. 1997;5:136-139, reported the diffuse aspect of chronic cellular inflammation, including macrophages and lymphocytes in the fibrous septa, from skin biopsies. According to this author, the septa would be responsible for mild inflammation resulting in lysis of adipocytes and skin atrophy. Others, however, find no evidence of inflammation or lysis of adipocytes in cellulitis (Pierard GE et al. Cellulite: from standing fat herniation to hypodermal stretch marks. Am J Dermatopathol. 2000;22(1):34-7; Scherwitz C & Braun-Falco O. So-called cellulite. J Dermatol Surg Oncol. 1978;4(3):230-4; Nurnberger F & Muller G. So-called cellulite: an invented disease. J Dermatol Surg Oncol. 1978;4 (3):221-9).

Thus, genetic, emotional, metabolic and hormonal factors, as well as age, sex, hypertension, obesity and habits such as smoking, sedentary lifestyle, tight clothing and poor diet predispose to the appearance of cellulite.

The authors also report that the format of cellulite is given by the apparent depressions, which are caused by the retraction of the skin through the subcutaneous fibrous septa, and the raised areas are also projections of subcutaneous fat.

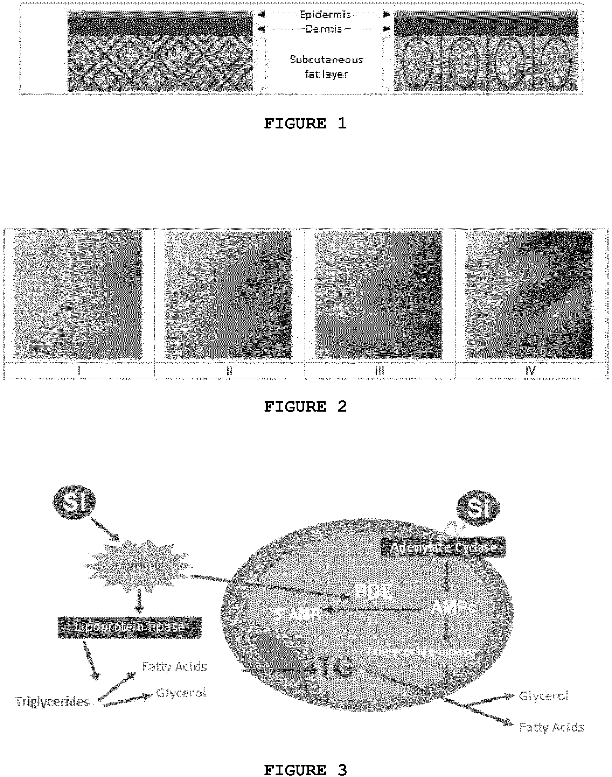

As cellulite affects women almost exclusively, the hormonal factor deserves attention in this direction. The distribution of cellulite in women which occurs in specific regions follows the same pattern as the deposition of adipose tissue. Biopsies show that, in women, there is a thin fibrous septum, with perpendicular projection, while in men, there is a thicker septum with oblique projection. (Figure 1). These histological features seem to favor the direction of the expansion of fat tissue, when increased toward the direction of depth in men, and toward the surface, in women.

Furthermore, the premenstrual period is related to worsening of the condition of cellulite. According to researchers, water retention and weight gain commonly occur in the premenstrual phase. The fluid retention may be due to a relative lack of progesterone and increased ovarian production of antidiuretic hormone (ADH) from the posterior pituitary gland.

Studies have observed that patients with cellulitis have increased regional or total body fat, which plays an important role in idiopathology. It is also known that sagging of the skin further aggravates changes in skin salience in most patients.

By analyzing the physiology of the mobilization of substances in the interstitium, involvement of both the venous and lymphatic systems was identified, in terms of drainage. If cellulite was exclusively a problem of the venous system, the use of diuretics would be sufficient for its treatment or minimizing of its effects. Thus, interference in the lymphatic system would be severe. However, when analyzing the lymphatic system it was observed that there is no evidence of obstruction associated with cellulite.

The key aspect is the presence of edema. In the association of edema, obesity and cellulite, the authors suggest treatment of the edema as a first step to ensure successful treatment.

One study concluded that mechanical factors can affect the appearance of cellulite, such as orthostatic changes and changes of the lumbar spine, such as hyperlordosis. In addition, there have been reports in the literature that in the areas where cellulite is evident, the skin's appearance changed depending on the position of the lower limb. Therefore, the authors concluded that body position tends to compress the fat in specific areas, thereby enhancing the appearance of the depressions (GB Milani et al. Correlation between lumbar lordosis angle and degree of gynoid lipodystrophy (cellulite) in asymptomatic women. Clinics (Sao Paulo). 2008,63(4:503-8).

Several forms of treatment are suggested in the literature, and a perceived consequence in reduction of the severity often leads to sagging skin (Rawlings AV. Cellulite and its treatment. Int J Cosmet Sci 2006;28(3):175-90; Avram MM. Cellulite: a review of its physiology and treatment. J Cosmet Laser Ther. 2004, 6(4):181-5; CF Sánchez et al. Thermography and histopathologic studies of cellulite. Rev Med Cosmiat Estet. 1994;1(II):3-13). It is impossible to establish the correct treatment without having a physiological understanding of the disease. Any treatment should be based on the attempt to reproduce the physiology of the organism.

Because it is a multifactorial aesthetic disorder, there are several treatments which involve a multidisciplinary team, where good results are obtained when the procedures and resources are seamlessly integrated.

To understand the process of formation of cellulite it is necessary to know its etiology, by studying the morphology and physiology of adipose tissue. Fat cells in the hypodermis, or adipocytes, which are cells with large vacuoles containing triglycerides, are grouped into lobes delimited by connective-vascular tissues. In these cells, fat storage and metabolism react only to hormones, and not to diet or physical activity.

It is known in the art that an increase in weight worsens the appearance of cellulite because it increases the volume in the fat layers (Smalls LK et al. Effect of weight loss on cellulite: gynoid lypodystrophy. Plast Reconstr Surg. 2006;118 (2):510-6). Opting for a healthy lifestyle, with a good diet and regular exercise, a good percentage of fat throughout the body may be decreased. This allows stored fat reserves to be metabolized by reducing their volume. Although cellulite cannot be effectively eliminated or reduced by diet or exercise, there may be a response to other recognized treatments.

Because it is a disease with significant aesthetic and psychological impact, there are numerous techniques that purport to solve the problem of cellulite.

A significant number of treatments are based on mechanical, manual or instrumental methods, in which skin-massaging seeks to increase blood circulation and lymphatic drainage, eliminating the accumulation of fluids and metabolites. Other techniques such as electrotherapy and iontophoresis are used for the treatment of cellulite, although there are doubts about their real benefit.

Another important segment, mainly from the standpoint of the cosmetic industry, is the treatment that uses active ingredients incorporated in creams, gels and the like. Topical delivery to active regions of interest, using appropriate formulations for the conduction of substances to the tissue, seeks to act on the metabolic mechanism of lipolysis.

Methylxanthines, such as caffeine, theophylline and aminophylline inhibit phosphodiesterase, thus providing β-adrenergic stimulation. The mobilization of triglycerides by these xanthines helps to locally transform the excess reserves of lipids into free fatty acids which are then eliminated by the lymphatic system.

Substances like silanols protect the tissue from the crosslinking of the fibers derived from non-enzymatic glycosylation, for example, caused by the action of oxygen free radicals, such as super peroxides and lipid peroxides. They may also modify the permeability of the venous and lymphatic capillaries to allow a more rapid removal of exudates.

Generally, the combined use of different methods of treatment, such as mechanical and cosmetic, combined with a balanced diet and regular exercise, tend to provide a significant improvement of cellulite.

A balanced diet reduces the amount of triglycerides stored in adipose tissue. Physical activity, in addition to consuming part of the stored fat, when performed correctly, provides an improvement in lymphatic and circulatory systems leading to more rapid and efficient elimination of metabolites and favors the balance of the lipogenic-lipolytic system. By providing improvement in the lymphatic circulatory system, β-adrenergic stimulants or β-adrenergic inhibitors have their effect increased.

Several forms of treatment are suggested in the literature with noticeable effects in reduction of severity. It is worth mentioning here that cellulite cannot be confused with obesity, where only adipocyte hypertrophy and hyperplasia occurs.

The endermology consists of a deep mechanical massage which improves the lymphatic system of the subcutaneous connective tissue, causing the fat cells to release excessive fat. However, a disadvantage is the time involved in repeated treatments, as well as the high cost (Marchand Privat Y. & JP; A new instrumental method for the treatment of cellulite. Au Femin Medicine.1991, 39: 25-34; Güleç AT. Treatment of cellulite with LPG endermology. Int J Dermatol. 2009;48(3):265-70; Collis NBS, Elliot LA, Sharpe C, Sharpe D. Cellulite treatment: a myth or reality: a prospective randomized, controlled trial of two therapies, endermology, and aminophylline cream. Plast Reconstr Surg. 1999;104(4):1110-4).

A cream and/or gel as cream containing aminophylline (advertised as a cosmetic for removing cellulite from the thighs) can be used as a topical treatment (Dickinson and Gora-Harper, "Aminophylline for Cellulite Removal," Vol 30, The Annals of Pharmacotherapy, p. 292-293, 1996). One drawback is that topical treatment with creams and/or gels leaves an oily residue (Wanner M, Avraham M. An evidence-based assessment of treatments for cellulite. J Drugs Dermatol. 2008;7(4):341-5; Avram MM. Cellulite: a review of its physiology and treatment. J Cosmet Laser Ther. 2004;6(4):181-5; Rossi ABR Vergnanini AL. Cellulite: a review. J Eur Acad Dermatol. Venereol 2000;14(4): 251-62; Rawlings AV. Cellulite and its treatment. Int J Cosmet Sci 2006;28:175-90).

There are some anti-cellulite creams that can help minimize the appearance of orange peel, for example, Lipo Peel®, from Vichy, which is a cream with abrasive cellulose particles, which serve to polish the skin and decongest the tissues; Cellu-Metric®, from Vichy, which is a program lasting 14 days that combats cellulite; Nivea body Gel-Cream Bye Bye Cellulite®, a cream that, if used regularly in the affected areas, promises to reduce the effect of orange peel and even promotes skin hydration; and Avon Renew Clinical Trilaser Corrector®, a cream that helps fight cellulite and stretch marks, by activating microcirculation and collagen production. However, all of these have the same drawback: it is necessary to wait for them to dry thoroughly before putting on the clothes, and they get on the clothing even if dry.

In terms of patent documents, there are several documents that describe various methods/formulations for the treatment or prevention of cellulite.

Some of these documents refer to compositions containing natural extracts, such as patent application KR20120137161, published on December 20, 2012, which contains as active ingredients Ricinus communis extracts (castor or ricin) and Sophora japonica (Japanese acacia); application CA2808197, published on February 23, 2012, which provides a method of treatment by applying a composition containing extract of Paulownia linters; document US2013052275, published on February 28, 2013, which discloses compositions containing herbs used in Ayurvedic medicine, such as cayenne, cinnamon, saffron, cumin, pepper, cardamom, ginger, fennel, cloves, mustard, paprika and garlic.

There are various patent documents related to devices used to improve the appearance of cellulite. Among them we can mention document WO2012019181, published on February 9, 2012; and document USD678543, published on March 19,2013.

Triacontanyl PVP is a type of plastic resin present in sunscreens. There is no product in the prior art for the treatment of cellulite using triacontanyl PVP in the composition. Therefore, it is still desirable to find a product for the treatment of cellulite by topical route which remains on the skin after application.

### Brief Description of the Invention

In the background of the invention, as described above, it can be seen that all of the types of cream or gel have the same drawback, which is the need to wait for the cream or gel to dry before putting on clothes. The present invention solves this technical problem with a composition comprising, as active ingredients, a compound formed by silanol, acefylline and alginic acid or a combination of an ester of salicylic acid (salicylamide) in polyoxyethylene, an alkaloid compound, an oil and/or plant extract, a natural antioxidant, and a film-forming polymer.

The composition of the present invention aims to lessen the appearance of cellulite. The application can be carried out in the process of aesthetic lymphatic drainage to assist in preventing the appearance of cellulite. Additionally, the present invention preferably utilizes a polymer regularly used in waterproof sunscreen, triacontanyl.

The first embodiment of the invention relates to providing a cosmetic composition comprising:
(a) from 4.0 to 27.0% by weight of active ingredients comprising:
   (i) at least one localized fat reducing agent selected from the group consisting of natural, modified and artificial substances,
   (ii) at least one alkali,
   (iii) at least one oil or vegetable extract,
   (iv) at least one antioxidant, and
   (v) at least one film forming agent;
(b) 6.0 to 32.0% by weight of cosmetically acceptable additives; and
(c) q.s.p. 100% by weight of hydrophilic gel-cream base.

Particularly preferred in the invention, is the cosmetic composition comprising:

| Ingredient (INCI names) | % (w/w) |
|---|---|
| Copolymers of sodium acrylates/lecithin | 1.30 |
| Propylene glycol | 5.0 |
| Disodium EDTA | 0.1 |
| Anhydrous caffeine | 1.0 |
| Pentylene glycol | 3.0 |
| Canola Oil | 1.6 |
| Dimethicone | 4.0 |
| Cyclomethicone | 3.0 |
| Perfume | 0.4 |
| Phenoxyethanol/Caprylyl Glycol | 0.8 |
| TEA Salicylate/ PEG-6/ Propylene Glycol | 1.10 |
| Acefylline methylsilanol mannuronate | 6.0 |
| Extract of Indian horse chestnut | 0.10 |
| Safflower Oil - Oil of *Carthamus tinctorius* | 0.5 |
| Menthol | 0.45 |
| CI19140 | 0.00125 |
| CI42090 | 0.00015 |
| Triacontanyl PVP | 2.0 |
| Nylon-12 | 1.0 |
| Simulgel INS-Copolymer of hydroxyethyl acrylate/sodium acryloyldimethyl taurate, isohexadecane, polysorbate 60 | 0.4 |
| Water | q.s.p.100.00 |

The second embodiment of the invention relates to the composition of the invention for the treatment, prevention or alleviation of gynoid lipodystrophy.

The third embodiment of the invention is a cosmetic product comprising said composition, said cosmetic product being preferably a gel-cream.

### Brief Description of the Figures

Figure 1 illustrates the structural difference in the subcutaneous fat layer between the sexes, male to the left and female to right.
Figure 2 illustrates the stages of cellulite according to the Nurnberger-Muller scale.
Figure 3 illustrates the mechanism of lipolysis.
Figure 4 illustrates the increase in cAMP and the relation to lipolysis.
Figure 5 shows the evaluated results in the reduction of measures with the application of a formula with Xantalgosil C.
Figure 6 illustrates the reduced volume of the observed zones after 30 and 60 days from the application of a formula with Xantalgosil C.
Figure 7 illustrates the chemical structure of the film-forming polymer (Antaron WP 660 - Triacontanyl PVP).
Figure 8 illustrates a graph of average temperature values of the skin surface taken before and after 28 days of use of the anti-cellulite composition (Mean ± standard deviation, n = 17).
Figure 9 shows a graph of the values of skin firmness, Ur/Ue, measured before and after 28 days of home use of the product (Mean ± standard deviation, n = 17).
Figure 10 illustrates a graph of the average result from the notes of the researcher for the "intensity of cellulite" parameter, at baseline and after 28 days of treatment in the study (Mean ± standard deviation, n = 17).
Figure 11 illustrates a graph of the average result from the notes of the researcher for "sagging" parameter, at baseline and after 28 days of treatment in the study (Mean ± standard deviation, n = 17).
Figure 12 illustrates a graph of the average result from the notes of the researcher for "swelling" parameter, at baseline and after 28 days of treatment in the study (Mean ± standard deviation, n = 17).
Figure 13 shows a graph of the average result of perceived sensory evaluation for efficacy at the start and after 28 days of treatment with the product under study.

### Detailed Description of the Invention

The present invention relates to a cosmetic composition for treating gynoid lipodystrophy comprising, as active ingredients: fat reducing agents such as a compound formed by silanol, acefylline and alginic acid and/or a combination of an ester of salicylic acid (salicylamide) in polyoxyethylene; an alkaloid compound; an oil and/or plant extract; a natural antioxidant and a polymer film former.

Differences in the accumulation of fat between the sexes are well known, in that the accumulation of fat in the male is compared to the apple shape, and in the female to the pear. This structural difference in fat accumulation also occurs in the subcutaneous layer as shown in Figure 1, with the representation on the left corresponding to the male and the female on the right.

Cellulite can be classified into four levels or phases in accordance with clinical and histopathological changes (see Figure 2):
- Level I: The patient is asymptomatic and shows no clinical change. Histopathology may show increased areal thickness, increased capillary permeability, micro-hemorrhages by diapedesis, capillary ectasia and fusiform microaneurysms within the post-capillary venules.
- Level II: After compression of the skin or after muscle contraction, there is a pallor and decrease of temperature and elasticity. Histopathologically, there is also peri adipocyte hyperplasia and hypertrophy associated with capillary dilation, micro-bleeding and an increase in the density of the capillary membrane.
- Level III: A padding of the skin and/or an appearance of "orange peel" is evident at rest; there is palpable sense of small granulations in the deep levels; pain to palpation; decreased elasticity; pallor and decreased temperature. Histopathologically: dissociation and thinning of the fatty tissues due to the formation of new collagen fibrils, followed by encapsulation of small collections of degenerated adipocytes, wherein micronodules are formed; sclerosis and thickening of the inner layer; dilatation of venules and small veins; formation of numerous microaneurysms and hemorrhage within the fat tissue; formation of new capillaries; obliteration of the boundary between the dermis and subcutaneous tissue followed by an increase in volume of fatty micronodules which are usually deformed; sclerosis and inclusion of adipocytes within the deep dermis tissue.
- Level IV: Has the same characteristics of grade III with more palpable, visible and painful nodules in the deep levels and an obvious wavy appearance of the skin surface. Histologically, the structural lobular fatty tissue disappears and some nodules are encapsulated by dense connective tissue. There is still diffuse lipoesclerose (followed by important microcirculatory alterations), telangiectasia, spider and varicose veins, and epidermal atrophy throughout the microscopic image.

The composition of the present invention comprises: (a) from 4.0 to 27% (w/w) of active ingredients comprising (i) at least one fat-reducing agent preferably selected from the group consisting of natural, modified and synthetic substances, (ii) at least one alkaloid, (iii) at least one plant or vegetable extract and/or oil (iv) at least one antioxidant, and (v) at least one film forming agent; (b) from 6.0 to 32.0% (w/w) of cosmetically acceptable additives; and (c) q.s.p. 100% (w/w) of a hydrophilic gel-cream base.

Preferably, the composition of the invention comprises (a) from 6,0 to 20% (w/w) of active ingredients comprising (i) at least one fat-reducing agent preferably selected from the group consisting of natural, modified and synthetic substances, (ii) at least one alkaloid, (iii) the least one plant or vegetable extract or oil, (iv) at least one antioxidant, and (v) at least one film forming agent; (b) from 13.0 to 22.0% (w/w) of cosmetically acceptable additives and (c) q.s.p. 100% (w/w) of a hydrophilic gel-cream base.

More preferably, the composition of the invention comprises: (a) from 10.5 to 11.0% (w/w) of active ingredients comprising (i) at least one fat-reducing agent, (ii) at least one alkaloid (iii) at least one oil and/or plant extract (iv) at least one antioxidant, and (v) at least one film forming agent; (b) from 17.0 to 18.0% (w/w) of cosmetically acceptable additives and (c) q.s.p. 100% (w/w) of a hydrophilic based gel-cream.

The fat reducing agents, according to the invention, include, but are not limited to, acefylline methylsilanol mannuronate (Xantalgosil®) ester of salicylic acid (salicylamide) polyoxyethylene (Cellulinol®), ivy extract (*Hedera helix*)/extract of Bilis/ polyoxyethylene ester of tartaric acid (Adipol®), collagen, *Huscus aculeatus,* butcher's broom extract or mixtures thereof. The most preferred fat reducing agents of the invention are acefylline methylsilanol mannuronate (Xantalgosil®) and/or salicylic acid ester (salicylamide) polyoxyethylene (Cellulinol®).

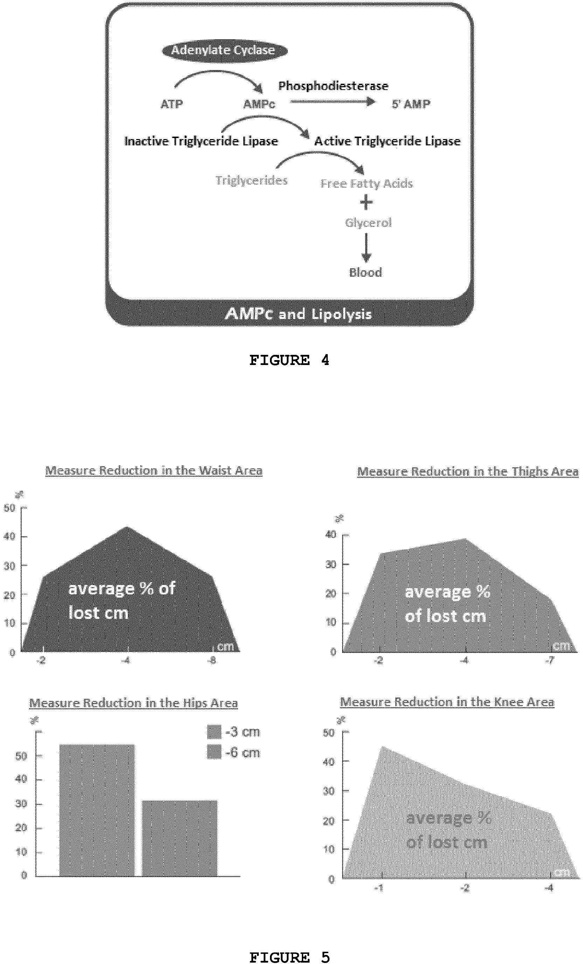

Xantalgosil C® is a silanol bound to alginic acid and acefylline, which acts by a dual mechanism on adipose deposits stimulating adenylate cyclase (increased cAMP) and by inhibiting phosphodiesterase, which activates lipase, an enzyme that breaks down fat, providing a rapid response in mobilizing the same. Figures 3 and 4 help to understand the action of the fat reducing agents.

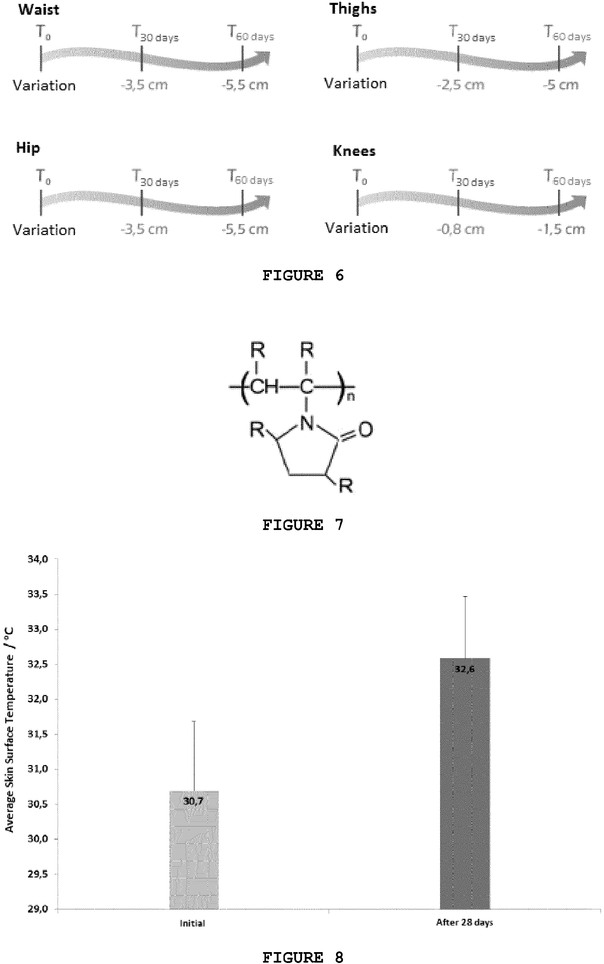

In a study of the activity of Xantalgosil C on localized lipodystrophy, the action of this product was clinically proven on dermo sculpture. 25 volunteers with varying degrees of cellulite were selected. They applied a formula of Xantalgosil C twice daily for one month. The inches lost in the waist, hips, thighs and knees were measured. The evaluated results in reducing measurements are summarized in Figure 5. The selected subjects were not overweight and had formation of cellulite in various parts of the body (hips, thighs, knees and arms), with a reduction of volume in observed areas. The results are shown in Figure 6.

The results verified that Xantalgosil C presents outstanding action in the activation of adipocyte lipolysis; global action, inhibiting the accumulation of fats and aiding in the elimination of fat already accumulated; increases tissue permeability and therefore the reabsorption of edema; inhibits degeneration of proteoglycans and mucopolysaccharides in elastogenic and collagenous fibers; stimulates the biosynthesis of collagen, helping to reverse the damage caused by the accumulation of lipids; moisturizes and restores tissues; and has rapid and profound action.

Another reducing agent especially preferred for use in the cosmetic composition of the present invention is the ester of salicylic acid (salicylamide) in polyoxyethylene, sold under the name Cellulinol®. This product is highly effective in penetrating the skin and is associated with anti-inflammatory, decongestant, astringent and keratolytic properties.

According to the invention, the alkaloids used in the cosmetic composition include, but are not limited to, methylated xanthines, e.g. caffeine, theophylline or aminophylline; atropine, quinine and mixtures thereof.

The especially preferred alkaloid for use in the cosmetic composition of the present invention is caffeine, more particularly, anhydrous caffeine. Caffeine is a methylxanthine with a high power of permeation of the skin, and acts deep in fat cells by promoting lipolysis - breakdown and burning of fat molecules (triglycerides), promoting reduction of measurement. It also operates in the dermis, stimulating the fibroblasts to synthesize collagen, elastin and other extracellular matrix components, leading to increased skin firmness. Additionally, it increases the levels of cyclic AMP in the cell by promoting proliferation by stimulating cellular metabolism.

Another important aspect in the cosmetic composition of the present invention is the presence of at least one oil and or plant/vegetable extract, which can be selected, without limitation, from the group consisting of *Aesculus hippocastanum* (horse chestnut extract), *Carthamus tinctorius* oil, arnica extract, Tamanu oil *(Calophyllum inophyllum* seed oil), mixture of Propanediol/Water/Extract of *Ruscus aculeatus* root, Extract of citrus medica limonum (lemon) bark / Extract of *Soligago virgaurea* / Extract of *Astragalus membranaceus* root (Drenalip®), seaweed extract, oat extract, calendula extract, *Asian centella* extract or *Centella asiatica* Extract, *Gingko biloba* extract, and mixtures thereof.

Particularly preferred for use in the composition of the invention is the extract of horse chestnut. The Indian nut *(Aesculus hippocastanum)* is a tree belonging to the Hippocastanaceae family (Wilkinsom JA et al. Chestnut-Aesculus Hippocastanum: Potential Applications in Cosmetic Skin-care Products. Int J Cosmet Sci 1999 Dec; 21(6):437-47; Hexsel D. et al. Botanical extracts used in the treatment of cellulite. Dermatol Surg. 2005, 31 (7 Pt 2):866-72). The dry extract obtained through its seed contains several active substances, but the main active ingredient is a mixture of triterpene glycosides, such as Escin. The main mechanism of action of the extract of Indian Chestnut is on reducing the activity of lysosomal enzymes, which are increased in chronic venous diseases, thereby inhibiting the decomposition of the glycocalyx (mucopolysaccharides) in the capillary wall region. Filtration of low molecular weight proteins, electrolytes and water into the interstitium is inhibited by reducing vascular permeability, promoting a reduction in the incidence of edema and an increase in venous tone. Thus, the Indian Chestnut has anti-inflammatory and anti-edema properties. The active Escin is capable of reducing lysosomal activity by up to 30%, in skin care products, probably by stabilizing the cholesterol content of the lysosomal membranes, thereby reducing the release of enzymes and capillary permeability.

The cosmetic composition of the present invention is distinctive, and unexpected in formulations known in the art, employing at least one film-forming polymer, with the objective of increasing the permanence of the gel-cream invention on the skin, and thus enables action on subject areas having the appearance of cellulite or areas where cellulite is already installed. According to the invention, the film-forming polymer used in the composition includes, but is not limited to, triacontanyl PVP, Bis-Diglyceryl Polyacyladipate-2 copolymer, PVP/MA copolymer, VP/Eicosene copolymer, PVP hexadecene copolymer, acrylate copolymer, acrylate/C12-22, alkylmethacrylate copolymer, and other cosmetically acceptable polymers and copolymers of acrylic acid and mixtures thereof.

Especially preferred for use in the composition of the invention is the film forming polymer Triacontanyl PVP, having the chemical structure shown in Figure 7, marketed under the name of Antaron WP 660®. It is added in the formula in order to increase the residence time of the active ingredients that act in the treatment of cellulite, enhancing the effectiveness of the product. The film formed by Antaron® WP 660 prevents the product from easily leaving the skin due to sweat or friction, and therefore acts as a patch, maintaining the active ingredients on the skin surface for a longer period and ensuring maximum performance of the formula.

Another important component of the composition of the invention is the antioxidant, selected from the group that includes, but is not limited to, tocopherol, lipoic acid, ascorbic acid, flavonoids, biflavonoids, resveratrol, and mixtures thereof.

Particularly preferred as the antioxidant ingredient of the composition of the invention is safflower oil (*Carthamus tinctorius).* This is a natural antioxidant that has properties which can accelerate fat metabolism, thereby assisting in the control of obesity. Studies indicated that this oil contains substances that act by causing the body to use stored fat as fuel contributing to greater fat elimination. This occurs because its nutrients can inhibit the action of a specific enzyme, lipoprotein lipase (LPL). The function of the LPL enzyme is to transfer the fat present in the bloodstream to the inside of fat cells, responsible for storing body fat and adipose tissue composing the human body. The higher and more intense the activity of this enzyme, the more fat is stored in fat cells and, as a consequence, causes weight gain. Therefore, safflower oil nutrients have the ability to block the action of LPL which causes the body to use existing stores of fat as an energy source, called lipolysis, which is the burning of fat.

Additives such as softeners, emollients, humectants, thickeners, sequestrants, preservatives, fragrances, dyes, sensory modifiers, cooling agents, emulsifiers, skin lightening compounds, and mixtures thereof, may also be employed in the composition of the invention.

Additional humectants include, but are not limited to, propylene glycol, butylene glycol, pentylene glycol, glycerin and mixtures thereof.

Additional emollients include, but are not limited to, canola oil, dimethicone, cyclomethicone, myristyl acid, palmitic acid, stearyl acid palmitate, lauryl lactate, myristyl, coco caprylate/caprylate oleate sterin, cetyl octanoate, sorbitol, butylene glycol and mixtures thereof.

Additional thickeners include, but are not limited to, acrylate copolymers of sodium/lecithin, copolymer of hydroxyethyl acrylate/sodium acryloyldimethyl taurate, isohexadecane, polysorbate 60, xanthan gum, carrageenan and mixtures thereof.

Additional sequestrants include, but are not limited to disodium EDTA.

Additional preservatives include, but are not limited to, phenoxyethanol/caprylyl glycol, esters of p-hydroxy benzoic acid, hydantoin derivatives, methyl paraben, propyl paraben, benzyl alcohol, and mixtures thereof.

The composition of the invention is preferably prepared in two steps or parts.

To avoid the negative effects of the presence of metallic ions in the composition of the invention, disodium EDTA is used as a sequestering or chelating agent. Furthermore, to make the cosmetic composition of the invention more attractive, scents and dyes such as CI19140 and CI42090, and a refreshing substance such as menthol, as well as a sensory modifier such as nylon-12, are added,

### EXAMPLES

The following, non-limiting examples illustrate the present invention.

### Example 1: Preparation of Cosmetic Composition of the Invention

A preferred composition of the invention comprises the following ingredients:

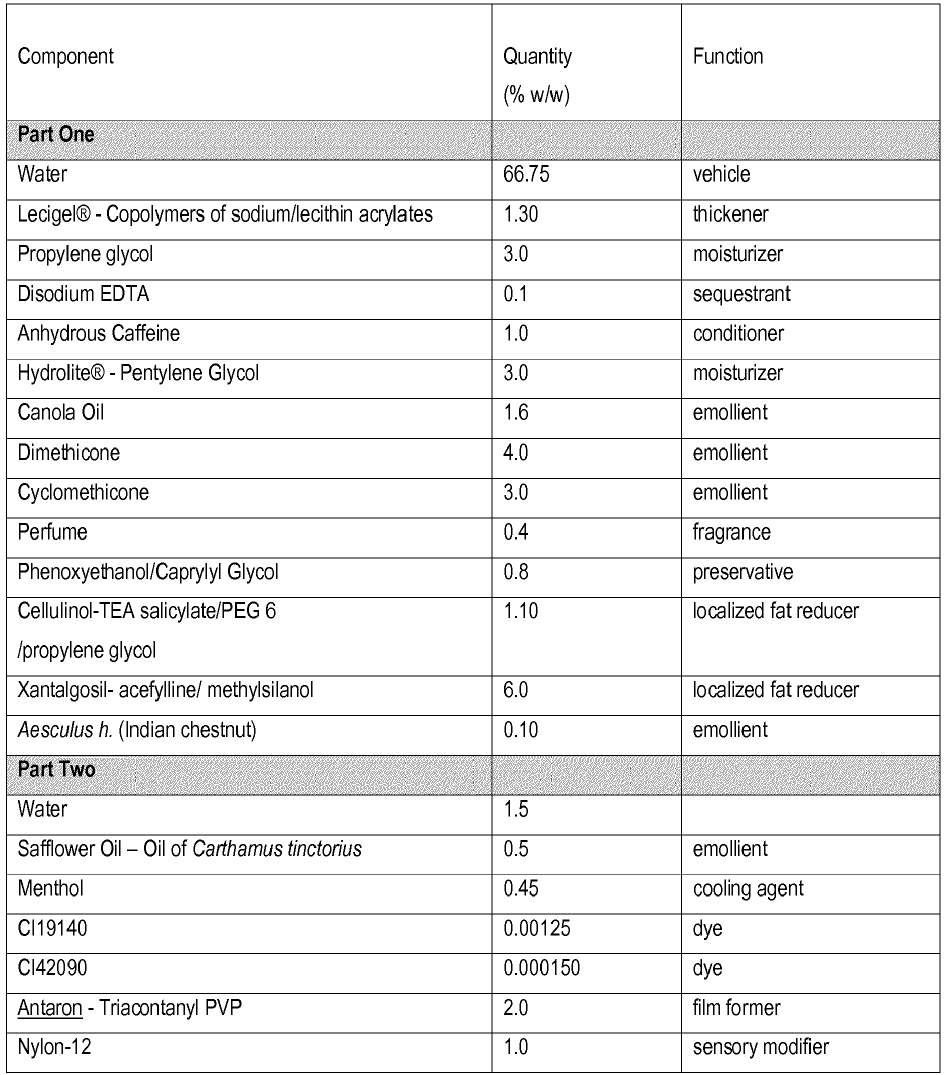

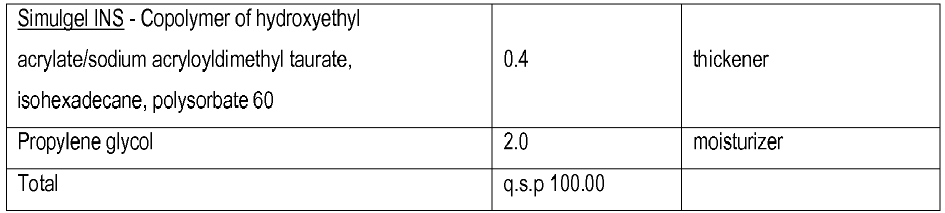

The above composition was prepared in two parts, using the methodology described below:
1 - In the main reactor, add the water, and, while stirring, add EDTA, caffeine, Lecigel® (gelling agent comprising soy phospholipids), propylene glycol, Hydrolite® (zeolite) and heat the mixture to 75-80° C.
2 - In the auxiliary heating tank heat the Triacontanyl PVP (Antaron WP 660®), canola oil enriched with phytosterols and natural anti-inflammatories (PreAct Lipex®) tocopherols and dimethicone, and heat to 75-80° C.
3 - Pour the mixture of item 2 over the mixture of item 1. Stir for 15 minutes while maintaining the temperature. Add the dyes.
4 - Start cooling to below 45°C and immediately add the cyclomethicone (DC 245®). When the temperature is below 45° C, add the phenoxyethanol/caprylyl glycol (Optiphen®), the TEA salicylate/PEG6/propylene glycol (Cellulinol®) and the safflower oil. Solubilize the menthol in the fragrance, and add to the product while stirring. Solubilize the Indian horse chestnut in the 2nd part of water and add to the mixture while stirring.
5 - Check the pH, which should be between 4.5 and 6.5 and add the acefylline methylsilanol mannuronate (Xantalgosil®).
6 - Add the nylon-12 (12-20 Tegolon®) dispersed in propylene glycol of the 2nd part. Adjust the viscosity with the mixture of hydroxyethyl acrylate copolymer/acrylodimethyl taurate, isohexadecane, polysorbate 60 (Simulgel INS®).
7 - Complete the weight with water.
8 - Measure pH and viscosity.

Specifications of the cosmetic composition of the present invention;
pH at 25° C = 5.6 to 6.2
Viscosity at 25° C = LVT/Spindle 3/0.6 rpm (1 min) -125 million to 141 million CP
Density at 25° C = 0.982 -1.002

### Efficacy Study of the Composition of the Cosmetic Invention

The anti-cellulite formulation was applied daily for 28 days by 18 volunteers.

The characteristics evaluated in this study were: increasing skin firmness via cutometer; reducing signs of gynoid lipodystrophy by evaluating the microcirculation via thermography; and reducing signs of gynoid lipodystrophy, by sensory analyses and perceived clinical effectiveness.

### Thermography

According to the test results, there was a significant average increase of the mean temperature of the skin surface of 6.2% after 28 days of treatment with the product sample under study. This indicated that the product caused a significant increase in microcirculation in affected regions of gynoid lipodystrophy by up to 7.5% after 28 days of use. The analysis results can be verified by the graph shown in Figure 8.

### Cutometer- Firming of the Skin

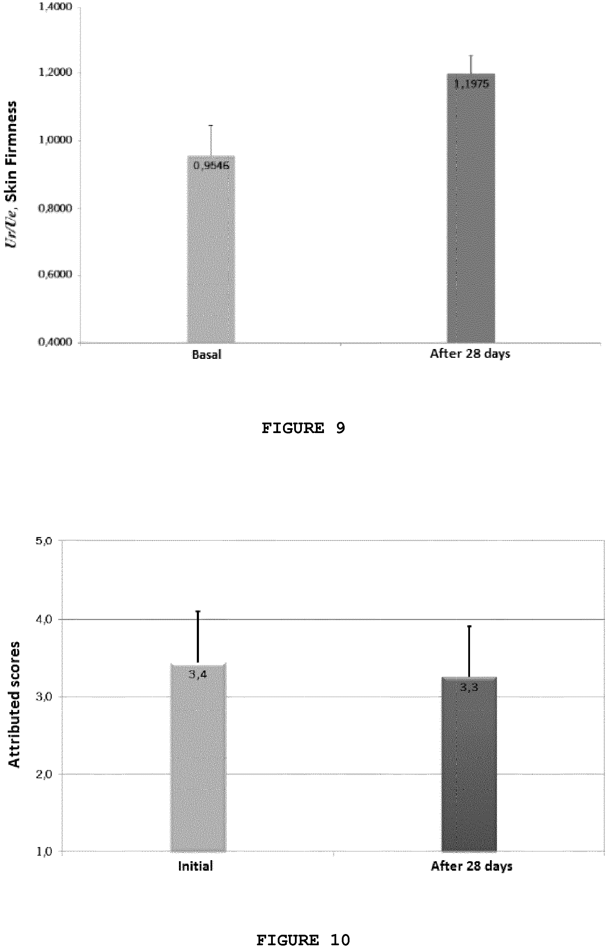

The product caused a significant increase in skin firmness of up to 33% after 28 days of use. The result was positive for 100% of the volunteers (Figure 9).

### Sensorial Evaluation of Clinical Efficacy

According to the analysis results for the "cellulite intensity" parameter after 28 days of treatment, there was a significant decrease in the mean score assigned, indicating a reduction in the "intensity of cellulite." The results of the parameter "cellulite intensity" were positive for 35% of the volunteers after 28 days (Figure 10).

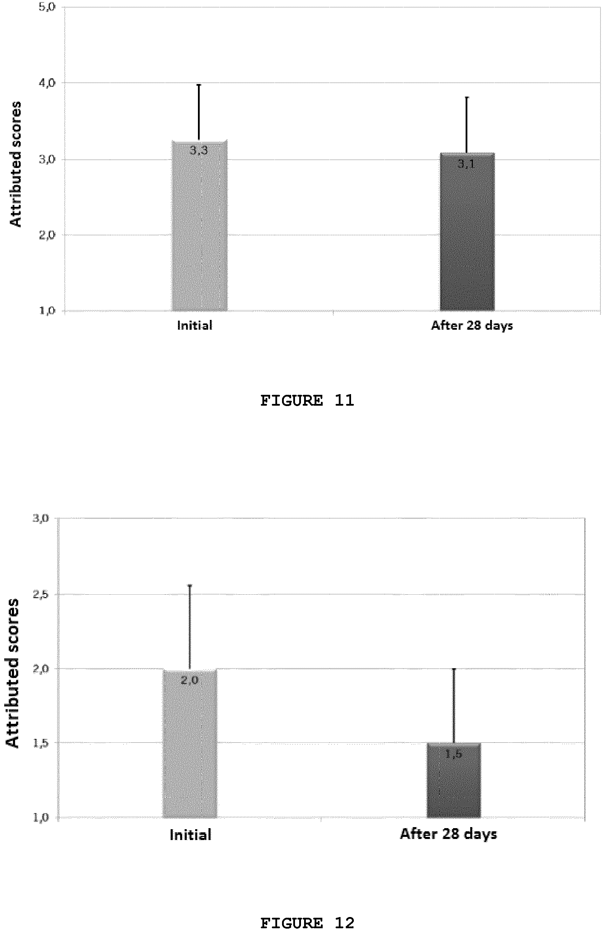

For the "sagging" parameter, after 28 days of treatment there was a significant decrease in the mean score assigned, indicating a reduction in the "sagging." The results of the "sagging" parameter were positive for 35% of the volunteers after 28 days (Figure 11).

For the "bump" parameter, after 28 days of treatment there was a significant decrease in the mean score assigned, indicating a reduction in "swelling." The results of the "bump" parameter were positive for 53% of the volunteers after 28 days (Figure 12).

### Sensory Evaluation by Perceived Efficacy and Acceptance

According to the results, it can be seen that:
- 100% of the volunteers realized that the product left the skin softer;
- 94% of the volunteers realized that the product left the skin more moisturized and improved overall appearance of the skin;
- 88% of the volunteers realized that the product reduced the appearance of cellulite, and left the skin more toned and smoother (less harsh);
- 82% of the volunteers realized that the product reduced swelling;
- 77% of the volunteers realized that the product left the skin (firmer) less saggy.

Furthermore, on average, the volunteers began to realize an improvement in visible cellulite, considering the aspect of "orange peel," after 12 days of treatment with the product.

With regard to pleasantness, it can be seen that:
- 100% of volunteers said that the product was easy to apply and spread and has a nice texture;
- 94% of volunteers approved the product;
- 88% of volunteers said that the product is easily absorbed by the skin and leaves a pleasant, silky touch;
- 77% of volunteers said that the product has a pleasant scent.

Regarding the intention to purchase, 94% of the voluntary participants said they would buy the product.

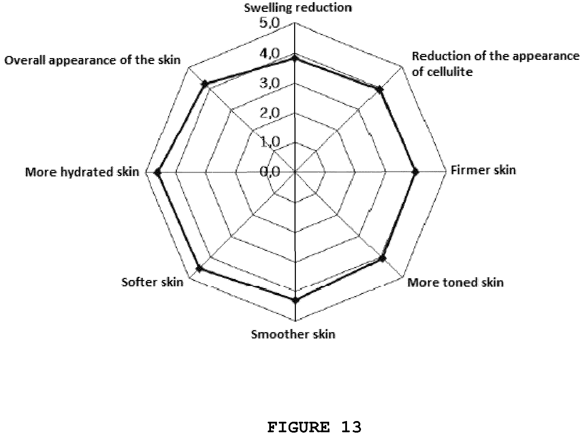

Results of the sensory evaluation of perceived efficacy and acceptance can be seen in Figure 13.

### Example 2: One of the preferred compositions of the invention comprises the following ingredients:

| Component | Quantity (% w/w) | Function |
|---|---|---|
| **Part One** | | |
| Water | 59.08 | |
| Lecigel® - Copolymers of sodium/lecithin acrylates | 1.70 | thickener |
| Propylene glycol | 3.5 | moisturiser |
| Disodium EDTA | 0.1 | sequestrant |
| Caffeine (57 to 100%), sodium salicylate, Lecithin, Silica | 8.77 | conditioner |
| Hydrolite® - Pentylene glycol | 3.0 | moisterizer |
| Safflower Oil | 1.6 | emollient |
| Dimethicone | 4.0 | emollient |
| Cyclomethicone | 3.0 | emollient |
| Perfume | 0.6 | fragrance |
| Phenoxyethanol/Caprylyl Glycol | 0.8 | preservative |
| Tamanu Oil(*Calophyllum inophyllum* seed oil, | 3.0 | localized fat reducer |
| tocopherol) | | |
| Xantalgosil - acefylline methylsilanol mannuronate | 6.0 | localized fat reducer |
| *Aesculus h.* (Indian chestnut) | 0.10 | emollient |

| **Part Two** | | |
|---|---|---|
| Water | 1.5 | |
| Safflower Oil- Oil of *Carthamus tinctorius* | 0.5 | emollient |
| Menthol | 0.50 | refreshing agent |
| PEG-40 Hydrogenated Safflower Oil | 0.90 | moisturizer |
| Octadecil di-t-butil-4-hidroxyhydrocinamate | 0.01 | antioxidant |
| Antaron® - Triacontanyl PVP | 2.0 | film former |
| Nylon-12 | 1.0 | sensory modifier |
| Simulgel INS - Copolymer of hydroxyethyl acrylate/sodium acryloyldimethyl taurate, isohexadecane, polysorbate 60 iso-hexadecane, polysorbate 60 | 1.35 | thickener |
| Propylene glycol | 2.0 | moisturizer |
| **TOTAL** | q.s.p.100.00 | |

All publications and patent applications mentioned in this specification are indicative of the level of those skilled in the art to which this invention pertains. All publications and patent applications are incorporated herein by reference to the same extent as if each individual publication or patent application were each specifically and individually indicated to be incorporated for ease of reference.

While certain embodiments have been described, they are only shown in an exemplary way, and are not intended to limit the scope of the invention. The claims that accompany this description and their equivalents are considered to cover such forms or modifications as they may be within the scope and spirit of the present invention.

## Claims

1. A cosmetic composition comprising:
(a) from 4.0 to 27.0% by weight of active ingredients comprising:
(i) at least one localized fat reducing agent selected from the group consisting of natural, modified and synthetic substances,
(ii) at least one alkaloid,
(iii) at least one oil or vegetable extract,
(iv) at least one antioxidant, and
(v) at least one film forming agent;
(b) 6.0 to 32.0% by weight of cosmetically acceptable additives; and
(c) q.s.p. 100% by weight of hydrophilic gel-cream base.

2. A composition according to claim 1 comprising:
(a) 6.0 to 20.0% by weight of active ingredients;
(b) 13.0 to 22.0% by weight of cosmetically acceptable additives; and
(c) q.s.p. 100% by weight of hydrophilic gel-cream base.

3. A composition according to claim 1, **wherein** said composition has at least one reducing agent for localized fat selected from Acefylline methylsilanol mannuronate (Xantalgosil®) ester of salicylic acid (salicylamide) polyoxyethylene (Cellulinol®), Ivy extract *(Hedera helix),* Bilis extract/ polyoxyethylene ester of tartaric acid (Adipol®), collagen, *Huscus aculeatus,* butcher's broom extract or mixtures thereof.

4. A composition according to claim 1, **wherein** said composition has at least one alkaloid selected from methylated xanthines, e.g. caffeine, theophylline or aminophylline; atropine, quinine and mixtures thereof.

5. A composition according to claim 1, **wherein** said composition has at least one oil and/or plant extract selected from *Aesculus hippocastanum* (horse chestnut extract), *Carthamus tinctorius* oil, arnica extract, Tamanu oil (*Calophyllum Inophyllum* seed oil), mixture of Propanediol/Water/Ruscus aculeatus root extract, Extract of citrus medica limonum (lemon) bark, extract of *Soligago virgaurea,* Extract of *Astragalus membranaceus* (Drenalip®), seaweed extract, Oat extract, calendula extract, *Centella asiatica* Extract, *Gingko biloba* extract, and mixtures thereof.

6. A composition according to claim 1, **wherein** said composition has at least one antioxidant selected from tocopherol, lipoic acid, ascorbic acid, flavonoids, bioflavonoids, resveratrol, and mixtures thereof.

7. A composition according to claim 1, wherein said composition has at least one film-forming agent selected from Triacontanyl PVP, bis-diglyceryl polyacyladipate-2 copolymer, PVP/MA Copolymer, VP/Eicosene copolymer, PVP hexadecene copolymer, acrylate copolymer, C12-22/Acrylates, ***alkylmethacrylate*** copolymer, and other cosmetically acceptable polymers and copolymers of acrylic acid and mixtures thereof.

8. A composition according to claim 1, **wherein** said cosmetically acceptable additives are selected from softeners, emollients, humectants, thickeners, sequestrants, preservatives, fragrances, dyes, sensory modifiers, cooling agents, emulsifiers, skin lightening compounds, and mixtures thereof.

9. A composition according to claim 8, **wherein** said humectant is selected from propylene glycol, butylene glycol, pentylene glycol, glycerin and mixtures thereof.

10. A composition according to claim 8, **wherein** said emollient is selected from canola oil, dimethicone, cyclomethicone, myristyl acid, palmitic acid, stearyl acid palmitate, lauryl lactate, miristyl, coco caprylate/caprate oleate stearin, cetyl octanoate, sorbitol, butylene glycol and mixtures thereof.

11. A composition according to claim 8, **wherein** said thickeners are selected from acrylate copolymers of sodium/lecithin, copolymers of hydroxyethyl acrylate/sodium acrylodimethyl taurate, isohexadecane, polysorbate 60, xanthan gum, carrageenan and mixtures thereof.

12. A composition according to claim 8, **wherein** said sequestrant is disodium EDTA.

13. A composition according to claim 8, **wherein** said preservatives are selected from phenoxyethanol/caprylyl glycol, esters of p-hydroxy benzoic acid, hydantoin derivatives, methyl paraben, propyl paraben, benzyl alcohol, and mixtures thereof.

14. A composition according to any one of claims 1-13 comprising the following formulation:
| Ingredient (INCI names) | % (w/w) |
|---|---|
| Copolymers of acrylates of sodium/lecithin | 1.30 |
| Propylene glycol | 5.0 |
| Disodium EDTA | 0.1 |
| Anhydrous Caffeine | 1.0 |
| Pentylene glycol | 3.0 |
| Canola Oil | 1.6 |
| Dimethicone | 4.0 |
| Cyclomethicone | 3.0 |
| Perfume | 0.4 |
| Phenoxyethanol/Caprylyl Glycol | 0.8 |
| TEA Salycilate/PEG 6/propylene glycol | 1.10 |
| Acefylline methylsilanol mannuronate | 6.0 |
| Extract of Indian Chestnut | 0.10 |
| Safflower Oil - Oil of *Carthamus tinctorius* | 0.5 |
| Menthol | 0.45 |
| CI19140 | 0.00125 |
| CI42090 | 0.00015 |
| Triacontanyl PVP | 2.0 |
| Nylon-12 | 1.0 |
| Simulael INS-Copolymer of hydroxyethyl acrylate/sodium acrylodimethyl taurate, isohexadecane, polysorbate 60 | 0.4 |
| Water | q.s.p. |
| | 100.00 |

15. Cream-gel formulation for the reduction of localized fat comprising:
(a) 4.0 to 27.0% by weight of active ingredients comprising:
(i) at least one localized fat reducing agent selected from the group consisting of natural, modified and synthetic substances,
(ii) at least one alkaloid,
(iii) at least one oil and/or vegetable extract,
(iv) at least one antioxidant, and
(v) at least one film forming agent;
(b) 6.0 to 32.0% by weight of additives; and
(c) q.s.p. 100% by weight of hydrophilic gel-cream base.

16. A composition according to any one of claims 1-15, **characterized in that** it is prepared in two steps or parts.

17. The composition according to claim 16 comprising the following formulation:
| Component (INCI names) | Quantity (% w/w) |
|---|---|
| **Part One** | |
| Water | 59.08 |
| Lecigel®-Copolymers of acrylates of sodium/lecithin | 1.70 |
| Propylene Glycol | 3.5 |
| Disodium EDTA | 0.1 |
| Caffeine (57 a 100%), sodium salycilate, lecithin, silica | 8.77 |
| Hydrolite® - Pentylene Glycol | 3.0 |
| Canola Oil | 1.6 |
| Dimethicone | 4.0 |
| Cyclomethicone | 3.0 |
| Perfume | 0.6 |
| Phenoxyethanol/Caprylyl Glycol | 0.8 |
| Tamanu Oil(Calophyllum Inophyllum seed oil, tocopherol) | 3.0 |
| Xantalgosil - Acefylline methylsilanol mannuronate | 6.0 |
| Aesculus h. (Indian Chestnut ext.) | 0.10 |
| **Part Two** | |
|---|---|
| Water | 1.5 |
| Safflower Oil - Oil of *Carthamus tinctorius* | 0.5 |
| Menthol | 0.50 |
| PEG-40 hydrogenated castor oil | 0.90 |
| Octadecyl di-t-butyl-4-hydroxyhydrocinnamate | 0.01 |
| Antaron® - Triacontanyl PVP | 2.0 |
| Nylon-12 | 1.0 |
| Simulael INS-Copolymer of hydroxyethyl acrylate/sodium acrylodimethyl taurate, isohexadecane, polysorbate 60 | 1.35 |
| Propylene Glycol | 2.0 |
| **TOTAL** | q.s.p.100,00 |

18. Composition according to any one of claims 1 to 17, for use in the treatment, prevention or alleviation of gynoid lipodystrophy.

19. Cosmetic product comprising a composition according to any of claims 1 to 18, said cosmetic product being a gel-cream.
